Europäisches Patentamt

European Patent Office

Office européen des brevets

(11) Publication number: **0 258 963**
A2

# EUROPEAN PATENT APPLICATION

(21) Application number: 87305026.4

(22) Date of filing: 08.06.87

(51) Int. Cl.⁴: **G01N 33/543** , G01N 33/558 , G01N 33/532 , G01N 33/76 , //G01N33/569

---

The title of the invention has been amended (Guidelines for Examination in the EPO, A-III, 7.3).

(30) Priority: 09.06.86 US 872355

(43) Date of publication of application:
09.03.88 Bulletin 88/10

(84) Designated Contracting States:
AT BE CH DE FR GB IT LI LU NL SE

(71) Applicant: ORTHO DIAGNOSTIC SYSTEMS INC.
One Johnson & Johnson Plaza
New Brunswick New Jersey 08933-7003(US)

(72) Inventor: Graham, Henry A., Jr.
RD2, Box 74 Sand Hill Road
Annandale New Jersey 08801(US)
Inventor: Mochnal, Dennis M.
18 Hancock Street
Clinton New Jersey 08809(US)
Inventor: Chang, Chi-Deu
60 Stella Drive
Bridgewater New Jersey 08807(US)
Inventor: Kang, Jemo
22 Nassau Court
Skillman New Jersey 08558(US)

(74) Representative: Jones, Alan John et al
CARPMAELS & RANSFORD 43 Bloomsbury Square
London, WC1A 2RA(GB)

---

(54) Colloidal gold immunoassay.

(57) A new immunoassay method for providing sensitive and rapid detection of ligands in body fluids utilizing colloidal gold labeled reagents and a matrix membrane having an immunologically active component therein. All liquid materials brought into contact with one side of the membrane flow through the membrane into an absorbent material in physical and fluid communication with the other side of the membrane. Reactions may be visually detected and in the case of hCG, a sensitivity of 50 mIU or less may be had within a total assay and manipulation time of one and a-half minutes.

## COLLOIDAL GOLD MEMBRANE ASSAY

### Field of the Invention

This invention relates to the field of immunoassays and more particularly provides a new immunoassay using colloidal gold and membranes for detecting hCG in urine and other body analytes in other body fluids.

### Background of the Invention

Many human health conditions can be ascertained by the use of immunologically-based immunoassays. Such assays rely upon the specificity of reaction between immunoglobulins, whether monoclonal or polyclonal-based, and their respective binding partners which may be haptens, antigens or other analytes all of which may hereafter be collectively and interchangeably referred to as ligands and ligand binding partners or biologically active molecules generally. Immunoassays are procedures which utilize the specific binding ability between ligands and ligand binding partners in determining the presence or absence of ligands or their binding partners in a fluid sample. Many such procedures are known and include sandwich asays, competitive binding assays and the like. While the instant invention utilizes the basic precepts of such assays, it is not deemed necessary to review in detail such conventionally known procedures.

Horisberger and Rosset, J. Histochem., Cytochem., 25:295-305 [1977], described an agglutination assay for mannan using colloidal gold as a label. While such colloidal gold agglutination assays have recently been commercialized, they have disadvantages associated with their speed, difficulty in determining subjective end points by color and the like.

It is an object of the present invention to employ colloidal gold as a label while avoiding disadvantages associated with Horisberger's agglutination assays.

In U.S. Patent No. 4,313,734, Leuvering describes a new immunoassay procedure involving the use of colloidal metal substances. While those procedures describe the use of a new label, i.e. colloidal metal, all were dependent upon the use of spectrophotometers or atomic flameless spectrophotometers for identifying the presence of the colloidal metal after it had been chemically leached from the site of immunological reaction. As a result, Leuvering's assays were dependent upon cumbersome procedures and expensive instrumentation in order to derive relatively insensitive results. It is not surprising therefore that the leaching procedures described by Leuvering have not found their way into the commercial marketplace which demands fast, sensitive and economical procedures capable of being performed without such dependence upon expensive instrumentation.

It is one aspect of the instant invention to utilize a colloidal gold label such as described by Leuvering but to utilize the label in an entirely new procedure which overcomes all of the deficiencies of the Leuvering procedures.

European Patent Application 0 158,746 and assigned to Janssen describes blot overlay assay methods which rely upon diffusion to the surface. While providing a dramatic improvement over Leuvering, the described methods still suffer from excessive time requirements to obtain minimally acceptable sensitivity. Janssen also described new techniques for improving the sensitivity or visualizability of assay results by innovative silver enhancement procedures.

Chun et al. reported in the 1986 Abstracts of the American Society of Microbiologists meeting (C-213 on Page 363 and C-409 on Page 396) the use of colloidal gold as a marker in assays using a format very similar to that discovered by Janssen. The Chun et al. assays, however, required significant incubation times (on the order of 2-3 hours) in order to get acceptable sensitivity.

It is an object of the present invention to provide yet additional improvements over the Janssen and Chun methods in sensitivity but most especially in the speed of assay.

In U.S. Patent No. 3,888,629, Bagshawe described an immunoassay procedure utilizing radioisotopes with a cartridge including a reaction surface in contact with an absorbent material for drawing fluids through the membrane. While the Bagshawe device claimed high sensitivity, it presented a less desirable solution inasmuch as it relied upon isotopic labels. Such labels require sophisticated detection equipment for determining their presence upon the reaction surface. Further, and as is well-known, radioisotopes pose substantial health and procedural difficulties making such procedures generally less desirable.

It is another aspect of the present invention to improve upon the mechanical aspects of the Bagshawe device while avoiding the disadvantages associated with isotopic procedures generally.

European Publication WO85/05451-A describes an apparatus for use in ligand receptor assays having a separating cellular membrane in contact with an absorbent material having capillaries in a direction perpendicular to the surface of the membrane or filter. While this large cylinder apparatus is substantially similar to that first described by Bagshawe, it relies upon limitations concerning the orientation of the absorbent fibers.

It is another aspect of the present invention to utilize a membrane and absorbent material combination while avoiding the manufacturing difficulties incurred by the specified orientation pattern given in the 85/05451 description.

It is yet another aspect of the present invention to provide assay procedures which may be interpreted entirely visually without reliance upon additional instrumentation.

It is yet a further aspect of the present invention to provide assays which may be produced economically, performed expeditiously, and derive the required sensitivity necessary to perform satisfactorily in all medical environments in both the stat and batch modes.

It is yet still another aspect to provide assays which can be visually read and which include internal controls which may form part of a positive result with a minimum of liquid reagents.

## Summary of the Invention

In keeping with the aspects and goals of the present invention, there is provided a new immunoassay procedure for determining the presence of ligands such as hCG in fluid samples such as urine. The assay is performed with a matrix surface having a tortuous path therethrough and having attached either the ligand or the ligand binding partner depending on whether a competitive or sandwich type assay is to be performed. In physical contact and fluid communication therewith is an absorbent material which acts to draw fluids through the matrix or membrane thereby bringing the fluid into contact with the ligand or ligand binding partner immobilized within the membrane. Thereafter, an additional ligand or ligand binding partner is contacted with the surface, such second immunologically active material being labeled directly or indirectly with colloidal gold. Ideally, an optional wash solution is then brought into contact with the membrane and it too is drawn through the matrix by the absorbent material, washing away unbound colloidal gold in the process. Surprisingly, colloidal gold remaining in the matrix by virtue of the presence of the ligand to be detected, such as by the conventional sandwich assay approach, results in the presence of a very strong, visually detectable colored spot.

As a result of the instant invention, fewer liquid reagents are required than in conventional assays, e.g. one reagent with an optional wash fluid as opposed to four or more reagents required with ELISA procedures. No instruments are required in order to read the results. And, the entire procedure including all handling steps and a single incubation step can be performed generally in three minutes or less, while still readily achieving great sensitivity. In the case of hCG, a sensitivity of 50 mIU or better is routinely obtained with a two drop sample and a total test performance time of approximately one minute, thirty seconds inclusive of incubation and handling times.

## Detailed Description of the Invention and Best Mode

The inventors hereof have surprisingly discovered that the unobvious combination of a matrix or membrane surface in contact with absorbent material, and immunoassays employing colloidal gold as a label, can result in extremely rapid and sensitive assays providing results which may be visually detected. Such an assay has not heretofore been possible. This is particularly true since surface assays such as those conducted on plastic simply fail to provide enough colloidal gold to be visually detectable. Surprisingly, a membrane surface provides many "layers" which unexpectedly capture enough colloidal gold in a form which is visually detectable.

The assay is most efficaciously performed utilizing a tortuous membrane filter bed or surface which permits the flow of fluids therethrough and to which biologically active molecules, most preferably, immunologically active components may be attached. By using such a tortuous membrane surface, reaction kinetics normally associated with solution phase reactions become possible despite the fact that one of the reactions takes place at a solid phase surface. While a number of types of filter membranes may be employed including for example Whatman 31 ET Chromatography Paper, nylon 66 such as BIODYNETM - (Pall Corp.), both ideally activated, nitrocellulose, and PVDF (polyvinylidine fluoride such as ImmobilonTM - (Millipore), the most preferred material is ImmobilonTM for ease of handling.

Using hCG (human chorionic gonadotropin) as an example of the utilization of the present invention in a sandwich assay format, one would attach hCG specific antibody onto the membrane. The membrane is preferably physically constrained in a holder so that it is in physical contact with an absorbent material. Such holders may take a variety of shapes but should preferably accomodate an amount of absorbent material sufficient to easily absorb the volume of liquids necessary to perform the assay and in a shape convenient for use by the clinician. Suitable absorbent material would include any of the so-called superabsorbents such as those used in diaper products, cellulose, cellulose acetate, rayon/cotton, polyethylene and polypropylene microporous plastic, glass microfibers, based on low cost and ready accomodation in manufacturing, any combination of cotton linter fibers such as Grade 470, GB003, GB004, or S300 from Schleicher and Schuell, the latter being most preferred.

Because the absorbents may be constrained to take virtually any shape, an impressive variety of mechanical devices may be envisioned for suitable presentation of the membrane. While small tubs such as the Icon arrangement (offered by Hybritech) come readily to mind, such are not the preferred embodiments inasmuch as those physical arrangements are bulky, difficult to store and overly large. It is thought Their size was necessitated by the large volumes of wash fluids required in their assay formats.

Because the assays of the present invention utilize colloidal gold, it was surprisingly discovered that such large volumes were no longer required to obtain the desired sensitivity with a visual signal. Accordingly, far greater freedom is obtained regarding the physical construction and preferable arrangements can no be employed. In particular, a slender dipstick is a preferred embodiment wherein the absorbent material is contained within a hollow intersticy of the dipstick and the membrane held in place at one end thereof. The top of the membrane is advantageously exposed for sample and reagent addition while the bottom of the membrane is in fluid communication with the absorbent material.

The fluid sample such as a urine sample is then applied to the exposed membrane and while the instant assay permits extraordinary variation in the volume, the most preferred sample volume is approximately two drops. It has been noted, however, that increased volumes, limited only by the absorbent capacity of the absorbent material, may be advantageously used to improve the assay's sensitivity. Other samples include, for example, whole blood, blood components, cerebrospinal fluid, aqueous discharges, saliva, solubilized discharges and the like.

Continuing with the hCG example, presence of hCG in the urine (typically associated with pregnancy) becomes attached within the membrane to the anti-hCG antibodies immobilized therein. The derivation of such antibodies, either of polyclonal or monoclonal nature is an art well-known and need not be described in detail here. The remaining portions of the sample including the fluid of the sample and non-hCG ligands are drawn, virtually immediately, through the matrix into the absorbent.

While one may now utilize an optional wash step to remove unbound material, the most preferred embodiment advantageously saves time and dispenses with such step as being unnecessary in order to obtain the clinically necessary sensitivities. Thus, the most preferred embodiment of the hCG assay utilizes as the first liquid reagent, a colloidal gold labeled antibody specific for a second epitope on the hCG or other ligand to be determined. Again, generally the most preferred immunoglobulins from a specificity and manufacturing viewpoint will be monoclonal antibodies derived through the methods of Kohler and Millstein first described in 1975 and published extensively elsewhere.

Experimentation has shown a substantial size range of colloidal gold may be utilized including from about 20 nm to about 92 nm with a preferred size within the range of 30 to 60 nm and the most preferred size approximately in the neighborhood of 40 to 50 nm. While 92 nm is the size (as determined on a Model 270 Submicron Particle Sizer available from Pacific Scientific) of the largest particle tested, there is no reason to believe larger particles, albeit more difficult to make, could not be used satisfactorily. Colloidal gold particles in the preferred size range may be visually observed as a reddish blue color when in solution. Preferred methods of colloidal gold formation and their attachment to immunoglobulins are described later.

While the best mode contemplates direct labeling of the ligand or ligand binding partner with colloidal gold, it is also contemplated that indirect labeling may be employed and even preferred in certain circumstances. Such indirect labeling would typically utilize a third immunoglobulin, which is itself labeled with the colloidal gold and is specifically reactive with the second immunoglobulin, in turn specific for the ligand to be detected. Such an approach is especially useful for antibody tests which employ colloidal gold labeled anti-globulin. Again, indirect labeling procedures are subjects readily understood by skilled practitioners in the art.

Following the sample addition in the most preferred embodiment, two drops of the colloidal gold labeled reagent are then placed onto the membrane (on the side opposing the absorbent material, as with all other solutions) and ideally given one minute to react. No special incubation conditions are required however, room temperature being perfectly adequate. Thereafter, two drops of a wash solution are ideally added in order to remove unbound colloidal gold material. In cases of strong reaction, e.g. where substantial ligand is present in the sample, it may not even be necessary to remove unbound colloidal gold by a wash in order to visually detect the reaction. Such a situation occurs for instance in the detection of 500 mIU/ml hCG which is present in the urine of pregnant women approximately three weeks after conception.

The colloidal gold membrane assay (hereafter COGMA) of the instant invention may be packaged in a kit which, in its simplest embodiment, contains the mechanical structure for containing the membrane and absorbent material and a vial containing the colloidal gold labeled ligand or ligand binding partner. Optionally, the kit may also contain the wash solution, a positive control for performing a control test, directions, or additional materials to permit performance of more than one assay.

It will be noted that as a result of the instant invention, fewer liquid reagents are required in order to perform an assay (indeed, most assays will require only one reagent having a biologically active component), and fewer steps need be performed. As a result, the typical reaction may be run in less than five minutes. often less than a minute and a half while still obtaining levels of sensitivity comparable to the best assays but, unlike those other assays, providing visually detectable, unequivocal results with astounding speed. Naturally, the invention is not limited to only visual detection but may also employ instrumentation to detect very weak reactions when only a limited amount of label is present. Such instrumentation would take advantage of the properties associated with colloidal gold such as its ability to scatter or block the passage of light and may also make the assay quantitative in nature if so desired.

Other advantages are provided by COGMA. Neither the reagents, nor the membrane containing a biologically active molecule need be at room temperature when the assay is performed. Indeed, the wash solution may nominally have a temperature of about 5-50°C with minimal sensitivity loss incurred at the extremes. This is in stark contrast with conventional ELISA procedures. Further, COGMA is not influenced to any practical extent by variations in the pH or ionic strength of the wash solution. Cold tap water may be effectively used as well as hot saline. Convenience of the operator may thus dictate the choice of wash solution.

The invention further avoids the disadvantages associated with ELISA techniques including the characteristic criticality associated with washing, timing, pH, and temperature. In contrast, the instant invention has no such limitations and indeed, providing the steps are performed in order, will perform even when executed carelessly. Indeed, the vast majority of pregnant women will give such strong positive results that performing a wash step will not be necessary in order to detect a positive result.

Perhaps the most surprising aspect of COGMA is the fact that a positive reaction may be visually detected at all. While this aspect of the invention is as yet still not fully understood by the inventors hereof, it is believed that the multi-layered nature of the matrix coupled with the light scattering properties of the colloidal gold results in accumulation, if not amplification, of the visual effect of the presence of colloidal gold.

In the most preferred embodiment, the visual effect of a positive reaction may be further enhanced by utilizing a readily distinguishable pattern. Patterns and hence reaction results have been found to be more readily identified by a practitioner when compared to a "spot".

Accordingly, the most preferred embodiment utilizes a distinguishable pattern such as a "plus" in a positive reaction wherein one of the crosses contains the anti-ligand binding partner, such as anti-hCG in the foregoing example. The other leg of the cross may advantageously be an antibody selected to react generally with the colloidal gold labeled antibody such as through an anti-species reactivity. For example, if the colloidal gold labeled reagent is a mouse monoclonal antibody, then the second leg of the "plus" might be a rabbit anti-mouse monoclonal antibody capable of reacting with all mouse monoclonal antibodies regardless of their individual specificities. Thus, only one reagent need be developed from a manufacturing perspective, and alteration of the assay procedure is avoided. These and other aspects of the internalized assay control is described in copending European patent application No. 87305028.0 (claiming priority from USSN 872 358-ORD 66 filed contemporaneously herewith.  ·

If the normal assay procedure is then followed, the result of the presence of hCG in the urine would be the appearance of a "plus" on the membrane surface. One leg of the "plus" would be attributable to the sandwich assay of hCG, the other leg attributable to the attachment of gold labeled monoclonal antibody by virtue of the immobilized anti-species monoclonal antibody or alternately, immobilized hCG-anti-hCG antibody complex. (While hCG alone would be sufficient for purposes of control, having the antibody present permits easier attachment of the hCG to the membrane). An additional advantage of this approach,

5

as more fully described in the co-pending European application referred to above,

is that the second leg of the "plus" having the anti-species antibody serves as a control to indicate that the antibodies present on the membrane are working and that the colloidal gold labeled reagent has been properly added. Both of these are requisite before that leg of the "plus" is detectable, in turn necessary for a positive reading. Absence of hCG in the urine results in a "minus" since only the anti-species antibody captures the anti-hCG colloidal gold labeled reagent. Further instruction in this regard may be obtained by direct reference to the above referred to application.

Other opportunities for use of COGMA should now become apparent. For instance, ovulation prediction is accomplished by monitoring a woman's urine for the presence of elevated LH (leutinizing hormone) content, indicative of imminent ovulation. Accordingly, the preferred embodiment of any LH ovulation test will incorporate a number of LH tests per kit; for example six tests to be run through days 9 through 14 for a 26-day cycle or days 11 through 16 for a 30-day cycle. The kit may also be expanded to 9 tests for those women suffering irregular menstrual cycles. Thus, a preferred mechanical arrangement would include a mechanical structure having absorbent material inside and exposing six membranes for sequential, daily use. The membranes may be in contact with the absorbent material, there being no critical need to provide separate absorbent materials for each well. Alternatively, other mechanical arrangements may provide for additional tests by providing additional membranes in a longer test pack. The most preferred kit components will then comprise the test pack including six to nine wells (membranes), liquid colloidal gold labeled biologically active molecule reagent, a urine collection cup and possibly a dropper for applying suitable reagents to each of the test wells. The kit may also optionally include a positive control, directions, and wash solutions in place of tap water.

Thus, the detection of LH would employ a membrane prepared substantially similarly as with hCG except that anti-LH antibodies will be used in substitution for anti-hCG antibody. The procedure is substantially as follows: 250 μls of urine sample is applied drop-wise onto the membrane in each well and the urine allowed to soak through (taking approximately 15 seconds). Thereafter, two drops of colloidal gold labeled anti-LH antibody is applied and allowed to soak through for approximately two minutes. Thereafter, one ml of tap water or the optional wash buffer is applied and the membrane examined visually for the presence of color, or, in the most preferred embodiment, for a "positive" sign indicative of the presence of LH in the sample.

In a number of instances, the presence of antibodies is used to determine exposure to disease causative organisms. Such tests include for instance Rubella IgG, Rubella IgM, Toxoplasmosis IgG and IgM, Cytomeglovirus IgG and IgM, HTLV III, Anti-EBNA, Mononucleosis, Anti-Core, HAA, Herpes, and Anti-Streptolysin O. Generically, antibody tests will be accomplished in substantially the same manner as the hCG test except however that in place of anti-hCG on the membrane, the ligand or antigen itself will be placed for reaction with the antibody in the patient sample. (Often, it is advantageous to attach antigen by way of antibody which is easier to attach.) The colloidal gold labeled reagent may then be suitably selected as an anti-human IgG or IgM as appropriate and labeled with colloidal gold. If the control portion of the membrane is human IgG, then that will serve as a control to capture the colloidal gold labeled reagent thereby providing, in the presence of the antibody to be detected, a "plus" as is most preferred. Absence of the antibody results in a "negative" which indicates that the colloidal gold labeled reagent was working and added appropriately, e.g. wash steps performed adequately in proper order. Absence of a "minus" indicates a failure of the test.

The assay is further suitable for those materials which cannot be employed in a sandwich assay because of the absence of multiple epitopic sites. In such instances, the present invention may be utilized with a conventional competitive aproach wherein the small molecule or hapten competes with a colloidal gold labeled reagent for a binding site on the membrane resulting in inhibition binding of the colloidal gold labeled material in the presence of hapten. Such haptenic materials could include for instance, theophyllin, drugs of abuse, hormones, digoxin, and the like.

For example, a small ligand such as THC in a sample would be added to a membrane having immobilized anti-THC and then washed. A liquid reagent comprising THC labeled with colloidal gold is added and then unbound material removed with a second wash step. Less gold will be visualized with increasing concentrations of THC in the sample. Alternatively, the hapten could be attached to the membrane. Separately the sample and an anti-ligand species 1 antibody is mixed and then put into contact with the membrane. Unbound material is washed away and then a second reagent comprising colloidal gold labeled anti-species 1 antibody is added. Similar results then obtain.

An assay for the detection of antibodies in patient fluid samples can be performed in the following manner. The antigen or ligand, for which the antibody is specific, is immobilized on and in the membrane and brought into contact with the fluid sample. The presence of the antibodies to be detected in the fluid sample are bound to their binding partner in and on the membrane. Unbound materials are preferably washed away and a reagent comprising colloidal gold labeled anti-human (or species of antibody to be detected) IgG bround into contact with the membrane. After a short, less than 5 minutes, incubation period and an optional wash, the presence of colloidal gold will be indicative of the presence of the antibody in the fluid sample.

The instant invention may also be employed to detect the presence of infectious disease agents including microorganisms such as <u>Streptococcus</u>, <u>Chlamydia</u>, <u>Gonococcus</u> or viral organisms such as hepatitis and the like.

Alternatively, an internal control could be provided by having an additional zone or bard of LH on the membrane strip. Optionally, the LH (or equivalent ligand) can be calibrated to known concentrations in order to provide a standard color comparative zone. Such internal comparative zones are additional novel aspects of the present invention. While one may now utilize an optional wash step to remove unbound material, the most preferred embodiment based on procedural simplicity advantageously saves time and dispenses with such wash step as being unnecessary in order to obtain the clinically relevant sensitivities.

These and other aspects of the present invention will become clear upon study of the accompanying examples.

Example 1 - Preparation of Colloidal Gold Sol

All glassware surfaces which come in contact with colloidal gold sold should be siliconized utilizing 1% Silwet 720 (Union Carbide) by soaking 10 minutes and then rinsing with Distilled Water (D.W.). 1 liter filtered distilled water is heated to 100°C. for at least 10 minutes. 10 ml of 1% gold chloride (Aldrich) in Milli-Q filtered D.W. is added to the reactor and mixed for one minute. 10 ml of 34 mM sodium citrate or 10 ml of 64 mM sodium malate or malic acid at pH 4.2 is added to the reactor to act as a reducing agent and rapid mixing continued for 20 minutes. A color change indicates successful formation of a gold sol. Heat source is removed and the reactor cooled to 15-30°C. 1 ml of 1% PEG 20,000 is added and pH of the sol and the reactor is adjusted to 7.1 ± .1 pH with the addition of 0.1 molar $K_2CO_3$. The colloidal gold O.D. may be measured at 540 and at 600 nm. In this form, the gold sol is suitable for coupling to antibody. Colloidal gold thusly produced will be approximately 40-50 nm.

Example 2 - Colloidal Gold--Antibody Coupling

Procedure 1 - Adsorptive Coupling

Antibody was dialyzed into 0.01 M HEPES buffer solution at pH 7.1 using 12,000-14,000 molecular weight cut off dialysis tubing and 300 ml solution per ml of antibody for 18 hours and then filtered through a 0.45 μm SWINEXTM filter. Protein concentration of antibody was obtained by measuring the antibody's absorption at 280 nm and diluted to a concentration of 3-4 mg/ml with 0.01 HEPES 7.1 to obtain optimal antibody--colloidal adsorption coupling as determined by the antibody to be labeled. With the sol present in the reactor from Example 1, the thusly filtered and diluted antibody was added while the sol was stirred at room temperature. Stirring continued for approximately 30 minutes whereupon (0.1 x sol volume) mls a buffer (D) comprising 0.01 M HEPES; 0.3 M D-MANITOL; 0.05% PEG 20,000; 0.1% BSA--RIA grade 0.05% sodium azide was added. Stirring continued at room temperature for 30 minutes. The solution was then transferred to a high speed centrifuge, centrifuged and the supernatent removed. The sol was then washed four times utilizing the aforedescribed buffer D and resuspended to 1/10 the starting sol concentration, filtered through a 0.45 μm membrane and stored in polypropylene at 2-8°C.

Preferred Procedure 2 - Covalent Coupling

Mix equivalent weight amount of BSA (100 mg BSA per 100 mg SOL) into SOL generated from previous step with pH preadjusted to 6.0. Stir for 2 hours at room temperature. Filter through 0.22 micron membrane filter to remove large particles. Measure OD 540 and 600 nm and calculate OD ratio; OD 540/OD 600 of acceptable material should be 2.50 ± 0.30. Take 200 ml of thusly prepared BSA sol, centrifuge (25,000 G for 30 minutes) and wash once with distilled water. Mix the washed particles with 1% wt/vol. glutaraldehyde and stir for 2 hours. Centrifuge (25,000 G for 30 minutes) and wash three times with distilled water. Mix the activated and washed particles with 2 mg of purified appropriate monoclonal IgG in pH 7.4 phosphate buffer and stir overnight at 2-8°C. Add 2.5 mg sodium borohydride to stabilize the coupling. Stir 30 minutes, and quench with 5 ml pH 8.0 glycine BSA buffer. Centrifuge (25,000 G for 30 minutes) and wash three times with phosphate buffered saline (pH 8.0). Resuspend and adjust dye concentration with final pH 8 buffer (50 mM triethanolamine, 100 mM NaCl, 0.2% BSA, 0.1% NaN$_3$) to about 10 O.D. at 540 nm. Filter through 0.22 micron filter and store at 2-8°C.

Example 3 - Membrane Prepraration

A PVDF membrane from Millipore (trade name Immobilon) was placed on a glass plate (TLC plate) and secure with tape for ease of handling. The surface was divided into one inch squares, marking the center of each square with a pencil. Using a LINOMAT™ applicator, approximately 2 μl/inch hCG-hCG complex or less preferably rabbit anti-mouse or hCG was applied vertically through the center marks, and anti-hCG antibody was applied horizontally through the center marks. The membrane was removed from the plate and incubated at room temperature for 30 minutes. Blocking solution comprising 2% w/v BSA and 0.5% w/v dry milk Type 1. 0.1% w/v TWEEN™ in distilled/deionized water was applied over the membrane and incubated for 90 minutes at 37°C. The membrane was washed with wash buffer comPrising 0.1% w/v TWEEN in TRIS 50 mM/NaCl 200 mM buffer at pH 7.5 for 15 minutes at room temperature. Thereafter the membrane was dried at 37°C. and stored in a moisture excluding container such as an aluminum or plastic bag containing desiccant at 2-30°C. until use.

Example 4 - Sensitivity For hCG In Urine

To a previously prepared membrane in contact with 100% cotton long linter fiber absorbent material having random fiber orientation, 250 μls of urine were added. Thereafter, 90 μls of colloidal gold labeled solution were added. After waiting one minute at room temperature, 2 mls of tap water were added and the results observed. The foregoing procedure was performed on urine containing the following known amounts of hCG:

### Sensitivity mIU hCG

|  | 0 | 5 | 10 | 25 | 50 | 500 |
|---|---|---|---|---|---|---|
| Observed Reaction | − | − | − | + | + | + |

Identical results were obtained when the test was repeated with a 30 second waiting or incubation period. The foregoing procedure was also repeated for urine samples from pregnant women and non-pregnant women with the following results:

|  | Number of Samples | Number Positive | Number Negative |
|---|---|---|---|
| Urines From Pregnant Women | 5 | 5 | 0 |

8

Urines From Non-
Pregnant Women          9                    0                    9

Example 5 - Altered Sample and Reagent Volumes

The procedures from Example 4 were repeated except 500 μls of urine and 150 μls of colloidal gold labeled reagent were employed with the following results:

## Sensitivity mIU hCG

|  | 0 | 5 | 10 | 25 | 50 | 500 |
|---|---|---|---|---|---|---|
| Observed Reaction | − | − | + | + | + | + |

Example 6 - Altered Membrane Sizes

A membrane prepared pursuant to the procedure of Example 3 and having dimensions of 3/8" x 5/8", was employed with the procedures of Example 4 except that two drops of urine (aproximately 40 μls) and two drops of colloidal gold labeled reagent (aproximately 40 μls) were used and three drops of tap water as a wash solution. Results were observed as follows:

## Sensitivity mIU (hCG)

|  | 0 | 50 | 100 | 500 |
|---|---|---|---|---|
| Observed Reaction | − | + | + | + |

The test was repeated with clinical samples:

|  | Number of Samples | Number Positive | Number Negative |
|---|---|---|---|
| Urines From Pregnant Women | 3 | 3 | 0 |
| Urines From Non-Pregnant Women | 7 | 0 | 7 |

Example 7 - Different Membranes

The procedures of Example 3 for membrane preparation were repeated for nylon 66 under the trade name of BIODYNE from Pall and a nitrocellulose obtained from Whatman Company. The filters were tested against spiked serum pools with 50 mIU hCG and negative urine pools. All results observed were positive for the spiked samples and negative for the negative urine pool.

As size of membrane is decreased, so is the volume of sample, colloidal gold labeled reagent, and wash (if employed) required. A membrane approximately half that of Example 6 would require only 1 drop per step.

It was noted that certain membranes and colloidal gold labeled reagents did not work well together and it is believed this may be due to deleterious charge effects. This may be advantageously avoided by simply choosing a different colloidal gold-membrane combination.

Example 8 - Procedure Using an Alternative Label to Colloidal Gold

hCG was attached to the BIODYNE membrane and serum containing mouse anti-hCG passed therethrough. Thereafter, Ortho FITC conjugated goat anti-mouse IgG (heavy and light chain) was brought into contact with the membrane and passed through into the absorbent material in contact therewith. The reaction was washed with phosphate buffered saline solution (PBSS) having 0.1% TWEEN 20. Placement of the membrane under an ultraviolet light indicated a fluorescent spot for the expected positive reaction. Serum containing 50 μgs per ml of antibody was positive while serum without antibodies was negative as observed by the absence of a fluorescent spot. (The ultraviolet light, an accessory piece of apparatus, is not required with the colloidal gold label.)

Example 9 - Membrane preparation for Ovulation Prediction Test

The procedure of Example 3 for membrane preparation was modified for membranes Biodyne™ (Pall Company), PVDF (Millipore Co.) and activated cellulose filter paper (Whatman) by the following procedure. Each membrane performed substantially identically.

The membrane was secured on the glass plate and the surface area was divided into 1.7 cm², marking the center of each square with pencil. Using the LINOMAT™ (CAMAG, Switzerland) applicator, approximately 2 μls/inch the hCG solution (300 IU/ml) was applied horizontally through the center marks, and anti-Beta-LH antibody was applied vertically through the center marks.

Alternatively, the coatings were accomplished using Schaffer fountain pens having the cartridge filled with hCG and anti-LH antibody, respectively. Straight lines were drawn on the membrane vertically with anti-LH antibody and horizontally with hCG solution using the filled fountain pen.

The membrane was then removed from the plate and incubated at room temperature for 30 minutes. Thereafter, the membrane was soaked in a blocking solution comprised of Tris-buffer with 2% BSA for 90 minutes at room temperature. The membrane was washed with Tris-Buffer containing 0.1% TWEEN™ 20 for 90 minutes at room temperature and then immersed in 0.5% aqueous polyvinylalcohol solution for 5 minutes at room temperature. Finally, the membrane was air dried at room temperature and stored in a desiccator at room temperature until use.

Example 10 - Sensitivity for LH in Urine

Tests for LH were performed following the procedure as described in Example 4 using Alpha-hCG specific antibody (which recognizes Alpha-LH), labeled with colloidal gold as prepared in Example 2, and membrane coated with Beta-LH specific antibody and hCG. The horizontal line coated with LH served as a negative confirmation and procedural test control and ensured that the test was performed properly and that the reagents were working. Thus, only those samples positive for LH would show a vertical line resulting in a plus sign (+). It will be noted, however, that one could vary the amount of LH equivalent of hCG on the control band (e.g. horizontal line in Example 9) to provide a specified color intensity or a predetermined cutoff level thereby providing an interal control for comparison to the test result (e.g. vertical line) by the user.

To a previously prepared membrane in contact with 100% cotton long linter fiber absorbent material, 150 μls of urine were added. Thereafter, 50 μls of colloidal gold labeled anti-alpha hCG antibody solution were added. After one minute, I ml of tap water was added and the results observed at any convenient time thereafter. The foregoing procedure was performed on urine standards containing the following known amounts of LH with the following results:

LH Concentration (mIU/ml)

| Standards | 0 | 5 | 10 | 25 | 50 | 500 |
|---|---|---|---|---|---|---|
| Sensitivity Observed | − | − | − | + | + | + |

The foregoing procedure was also repeated for urine standards containing the following known amounts of LH:

LH Concentration (mIU/ml)

| Standards | 0 | 5 | 15 | 40 | 100 | 200 |
|---|---|---|---|---|---|---|
| Observation | − | − | − | + | + | + |

The foregoing procedure was repeated for urine samples from two women having a 28 day menstrual cycle. The samples were collected over a five day period. The test results were verified by Advanced Care's Ovutime Test™ (Ortho Pharmaceutical Corporation) as follows:

Sample 1

| Day in cycle | 9 | 10 | 11 | 12 | 13 |
|---|---|---|---|---|---|
| Test Result (actual sign) | – | – | + | + | – |
| Ovutime Result | – | – | + | + | – |

Sample 2

| Day in cycle | 9 | 10 | 11 | 12 | 13 |
|---|---|---|---|---|---|
| Test Result (actual sign) | – | – | + | + | – |
| Ovutime Result | – | – | + | + | – |

Example 11 - Membrane Preparation for Rubella Antibody Test

The procedure of Example 9 for membrane preparation was modified as follows:
Activated cellulose filter paper (Whatman 31 ET) was cut into circular discs, each having 27 mm diameter. The membrane was placed on the glass plate and the center of each membrane was marked with a pencil. Application of biologically active molecules was accomplished using two Schaffer fountain pens having the cartridge filled with Rubella antigen and human immunoglobulin, respectively. Straight lines were drawn on the membrane vertically with Rubella antigen and horizontally with human immunoglobulin.

The membrane was then removed from the plate and incubated at room temperature for 30 minutes. Thereafter, the membrane was soaked in a blocking solution comprised of Tris-buffer with 5% BSA for 90 minutes at room temperature. The membrane was washed with Tris-buffer containing 0.1% TweenTM 20 and then immersed in 0.5% aqueous polyvinylalcohol solution for 5 minutes at room temperature. Finally, the membrane was air dried at room temperature and stored in a desiccator at room temperature until use.

Example 12 - Assay for Rubella Antibody in Serum

Tests for Rubella immunoglobulin in serum were performed using the membrane coated in Example 11 and colloidal gold labeled mouse anti-human immunoglobulin as prepared in Example 2. The horizontal line coated with human immunoglobulin served as a negative confirmation and procedural test control to ensure that the test was performed properly including adequate washing and that the reagents were working. Thus, only those samples positive for Rubella antibody would show a vertical line resulting in a plus sign ( + ).

To a previously prepared membrane in contact with 100% cotton long linter fiber absorbent material, 50 $\mu$l of sample diluted in 300 $\mu$l of PBS buffer containing 5% BSA and 0.05% Tween 20 (buffer A) was applied. The membrane was incubated for 2 minutes at room temperature, followed by washing with 1 ml of buffer A. Thereafter, 200 $\mu$l of colloidal gold labeled anti-human immunoglobulin solution was added. After one minute, 1 ml of tap water was added and the results observed at any convenient time thereafter. The foregoing procedure was performed on serum standards and serum samples collected from known individuals previously tested by conventional ELISA methods.

|  |  | Reference (ELISA) | Test Result |
|---|---|:---:|:---:|
| 1. | Positive control | + | + |
| 2. | Negative control | − | − |
| 3. | Sample 1 | − | − |
| 4. | Sample 2 | + | + |
| 5. | Sample 3 | + | + |
| 6. | Sample 4 | + | + |

The foregoing test was also repeated with Biodyne membrane prepared as in Example 11 and the same results observed.

From the foregoing, it will be readily perceived by one skilled in the art that numerous alterations may be made with regard to the selection of immunologically active components, the membranes, and the liquid reagent while still employing the essence of the invention comprising the combination of a colloidal gold labeled reagent in an immunoassay performed in a matrix membrane by flowing materials through the membrane into absorbent or other hydrophilic material in physical contact with the membrane to obtain a visually detectable result. For example, multiple spots or multiple antigens may be applied to the membrane such as for hepatitis or AIDS antibody detections whereby any positive will serve as a disqualifier for bloodbanking.

### Claims

1. A method for detecting a ligand in a sample comprising the steps of:

a) providing a porous membrane having a tortuous path therethrough and further having attached thereto a ligand binding partner, said membrane having a first side and a second side, said second side being in physical contact with an absorbent material for absorbing fluids brought into contact with said membrane, whereby fluid communication therebetween is established.

b) contacting the first side of said membrane with said sample and, simultaneously or successively, with a ligand binding partner or ligand labelled directly or indirectly with collodial gold whereby an immunological reaction is carried out; and

c) observing said first side of said membrane for the presence of colour indicative of the presence of colloidal gold and of the ligand in the sample.

2. The method of claim 1, wherein:

the ligand is an antigen having at least two epitopic sites;

the ligand binding partner in step a) is an immunoglobulin specific for a first epitopic site on the antigen; and

the ligand binding partner in step b) is an immunoglobulin specific for a second epitopic site on the antigen, the second immunoglobulin being labelled directly or indirectly with colloidal gold.

3. The method of claim 2, wherein the antigen is hCG.

4. The method of claim 1, wherein:

the ligand is an antigen;

the ligand binding partner in step a) is an immunoglobulin specific for the antigen; and

the ligand in step b) is a ligand, labelled directly or indirectly with colloid gold, which competes with the ligand in the sample and which is applied simultaneously with the sample.

5. The method of claim 1, wherein:

the ligand is an antibody;

the ligand binding partner in step a) is an antigen for which the antibody is specific; and

the ligand binding partner in step b) is an immunoglobulin, labelled directly or indirectly with colloidal gold, which is specific for the antibody.

6. The method of any one of claims 1 to 5, wherein, after the application of the ligand or ligand binding partner, labelled directly or indirectly with colloidal gold, a wash solution for removing unbound colloidal gold is applied to the first side of the membrane.

7. The method of any one of claims 1 to 6, wherein said colloidal gold has a size within the range of 20 to 92 nm.

8. The method of claim 7, wherein said colloidal gold has a size within the range of 30 to 60 nm.

9. The method of any one of claims 1 to 8, wherein said absorbent material is cellulose, cellulose acetate, rayon/cotton, polyethylene microporous plastic, polypropylene microporous plastic, glass microfibers, or cotton linter fibers.

10. The method of any one of claims 1 to 9, wherein the membrane comprises activated Nylon 66, nitrocellulose or activated polyvinylidine difluoride.

11. The method of any one of claims 1 to 10, wherein:

a ligand having specificity equivalent to the ligand in the sample is also attached to the membrane; and

the equivalent ligand and the ligand binding partner in step a) form a pattern which is visually detectable if the ligand is present in the sample and the ligand binding partner in step a) is working.

12. The method of any one of claims 1 to 11, wherein said membrane is incubated for a period of less than five minutes after it has been contacted by the sample.

13. The method of any one of claims 1 to 12, wherein the entire method is performed in a time period equal to the incubation period, if any, and the time required for the contacting steps, the latter generally totalling less than about 30 seconds.

14. The method of any one of claims 1 to 13, wherein said membrane further comprises a built-in calibrator for said ligand to be detected.

15. A kit for use in an assay for the detection of a ligand in a fluid sample comprising: a membrane having a ligand binding partner for binding to the ligand to be detected in a first zone; an absorbent material in fluid communication with said membrane for absorbing fluids brought into contact with said membrane; and a container having a liquid reagent comprising colloidal gold labelled ligand for binding to the ligand binding partner on the membrane in a competitive assay or to the ligand in the fluid sample in a sandwich assay.

16. The kit of claim 15, wherein said membrane has a second zone capable of binding with the liquid reagent.